# EUROPEAN PATENT APPLICATION

(11) **EP 1 306 793 A2**
(43) Date of publication of application: **02.05.2003**
(21) Application number: 02255937.1
(22) Date of filing: 27.08.2002
(51) Int. Cl.: G06F 19/00

(54) **Remote health-monitoring system and method**

(30) Priority: 24.08.2001 US 314739 P
(71) Applicant: March Networks Corporation, Ottawa, Ontario K2K 2X3 (CA)
(72) Inventor: The designation of the inventor has not yet been filed
(74) Representative: Maggs, Michael Norman

(57) **Abstract**

The present invention is directed to a remote health-monitoring system and method for the remote monitoring and supervision of outpatient vital signs using videoconferencing techniques. The system includes a management site, at least one medical professional site, a patient site, and a computer program operative to facilitate communications between the management site, the medical professional site, and the patient site to provide remote health monitoring. Video images and physiological data of a patient are digitally transmitted from a patient to a remote health care provider over a communications network, typically the Internet.

## Description

### Field of the Invention

The present invention relates generally to home health care systems, and more particularly to a remote health-monitoring system and method.

### Background of the Invention

The concept ot home health care began in the 1850's when traveling health care professionals would provide in-home visits to patients in need of health care, yet unable to seek such care on their own. From the outset, however, the home health care provision suffered from the problem of "downtime" from having to travel to a patient's home to deliver the needed service. Today, this problem has been compounded by the shortage of health care professionals providing home health care and by rising medical costs. In fact today it is often difficult, if not impossible, for a health care professional to justify the costs of performing home health care visits.

Paradoxically, while physicians now commonly monitor a patient's well being via health parameter measurements made during regularly scheduled office visits, the relentless pressure to reduce costs in the health care industry has required the more efficient use of a health care professional's services. During recent years, steadily increasing healthcare costs and outpatient populations have created a need to maximize time intervals between office visits. As a result, a number of vital health monitoring functions, traditionally performed by nurses and physicians, are now more often prescribed as a patient self-care responsibility. Large numbers of physicians now regularly prescribe home monitoring of such health parameters as blood pressure, heart rate, blood glucose level, clotting factor, body sounds produced by a stethoscope, EKG (electrocardiogram) signals, blood pressure, and artificial heart valve clicks.

Home health care systems have been proposed that allow the transmission of a patient's physiological data from their home to a health care professional at a remote location over a communications network. One common method involves the use of videoconferencing in which two or more people are connected audio-visually over a telephone line or other suitable two-way communications channel. Teleconference visits can be used to check up on patient recovery progress, verify medication compliance, illustrate to a patient how to perform home care, and the like.

The problem is that these systems are often inconvenient and/or inefficient. The home medical sensors are typically attached to the outside of a host unit, and either dangle there or are wrapped around the device with no integrated cable management offered. Medical sensors are typically wired to the circuit boards that read or drive them. Their wires are easily tangled, especially on monitors with multiple sensors. This tangled mess of cables is not user friendly, is obtrusive, and lacks discretion and privacy. As well, the sensors are generally fixed elements and therefore cannot be tailored to individual patients and their changing medical conditions. What is needed is a system where sensors can be quickly and easily interchanged or customized.

In addition, in existing remote patient monitoring systems, the vital sign-monitoring component of the system is required to be hard-wired to the host. This limits where it can be positioned in relationship to the host. As well, existing systems are not battery powered, generally requiring them to be plugged into the wall to provide a power source. This is less desirable since it again limits the unit's portability.

As well, in current IP based communications systems, an IP address is used to identity a client's computer or gateway. Currently, Internet Service Providers (ISPs) do not provide a static IP address to their customers, but rather, assign them dynarnically. The implications of this are that the address on the network of the patient's gateway computer 52 or the nurse's computer can change on a regular basis. This would also happen if the nurse accesses the system from both work and home computers.

Another problem is that the use of a camera in the home can raise user concerns over privacy and discretion. Having an exposed video camera, even when turned off, may raise the concern that the user is being monitored continuously. While shutters have been used on video cameras in the past for video conferencing applications, these shutters merely cover the lens of the camera. The camera is neither disguised nor protected in the process. Cameras have also been disguised in the past for surveillance or security purposes. Spy cameras are hidden in common objects such as a book in a bookcase, but typically use pinhole cameras that provide very poor quality video images. Security cameras can be hidden behind smoked glass or a one-way mirror but need to be professionally installed, adding cost to the system. For the home health care market, it is desirable that when the device is not in use, that its intended purpose is not evident when the unit is closed.

For the foregoing reasons, there is a need for an improved method and system for the provision of home health care.

### Summary of the Invention

The present invention is directed to a remote health-monitoring system and method. The system includes a management site, at least one medical professional site, a patient site, and a computer program operative to facilitate communications between the management site, the medical professional site, and the patient site to provide remote health monitoring.

The management site has a remote health-monitoring application server and a database accessible by the application server. The medical professional site has a client computer and a first videoconference camera in communication with the client computer. The patient site has a gateway computer, a second videoconference camera in communication with the gateway computer for relaying images of a patient, a medical kit having physiological data measuring devices in communication with the gateway computer for relaying a patient's physiological measurements, and a television for viewing informational displays. In an aspect of the invention, the medical kit is in wireless communication with the gateway computer.

The method includes the steps of managing remote health-monitoring using a remote health-monitoring application server; and a database accessible by the application server, visiting a patient remotely using a client computer and a first videoconference camera in communication with the client computer, receiving a remote visit using a gateway computer, a second videoconference camera in communication with the gateway computer for relaying images of a patient, a medical kit having physiological data measuring devices in communication with the gateway computer for relaying a patient's physiological measurements, and a television for viewing informational displays, and facilitating communications between all elements to provide remote health monitoring using a computer program.

A remote visit is a convenience to the patient, especially for those patients who would have difficulty traveling to the doctor's office or hospital. A videoconference visit, connecting a nurse station to a patient station via a communications link, is more economical than a home visit, thereby providing both convenience and cost savings.

Other aspects and features of the present invention will become apparent to those ordinarily skilled in the art upon review of the following description of specific embodiments of the invention in conjunction with the accompanying figures.

### Brief Description of the Drawings

These and other features, aspects, and advantages of the present invention will become better understood with regard to the following description, appended claims, and accompanying drawings where:
Figure 1 is an overview of a remote health-monitoring system in accordance with the present invention;
Figure 2 is an overview of a remote health-monitoring method in accordance with the present invention;
Figure 3 illustrates the infrastructure architecture of an embodiment of the system;

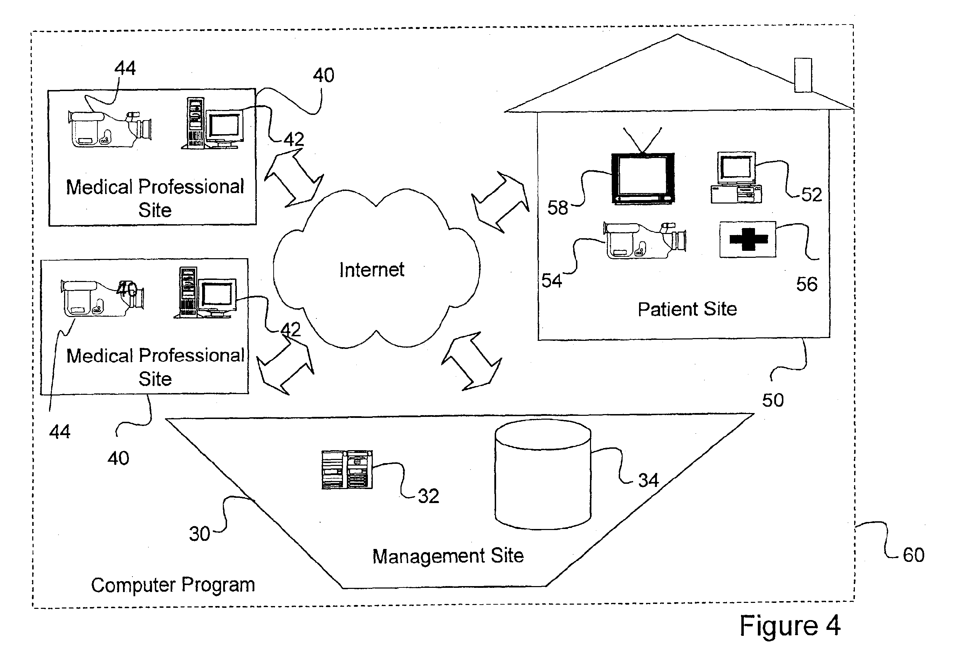

Figure 4 illustrates an embodiment having an additional doctor site;
Figure 5 illustrates a modular medical Kit;
Figure 6 illustrates a modular medical kit;
Figure 7 illustrates an interior panel of the medical kit;
Figure 8 illustrates a schematic layout of the medical kit;

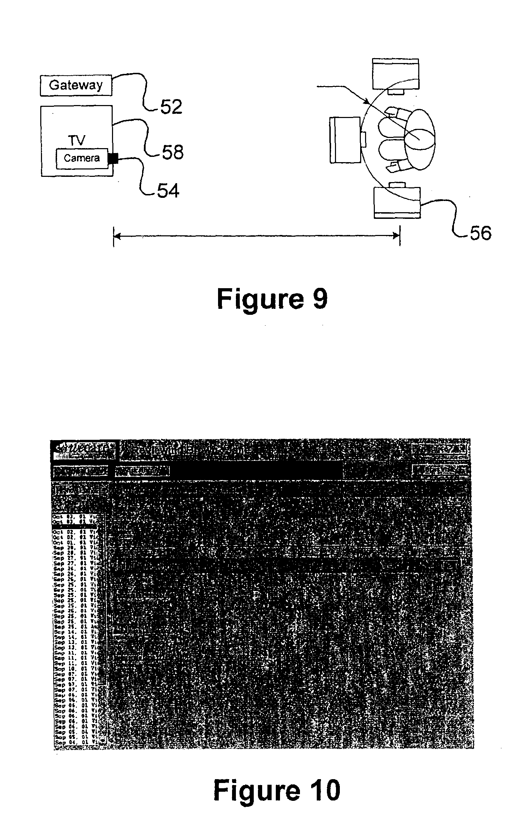

Figure 9 illustrates a physical arrangement of the system components in a residence;
Figure 10 illustrates a user interface;
Figure 11 illustrates a user interface;
Figure 12 illustrates a vital sign history tab;
Figure 13 illustrates all vital sign readings for a particular client;
Figure 14 illustrates a record history tab containing all medical records for a client;
Figure 15 illustrates a record history tab listing all sound tracks and their captions;
Figure 16 illustrates a nurse view tab;
Figure 17 illustrates a client view tab showing all the clients within a branch; and
Figure 18 illustrates a nurse availability chart tab showing time availability status of all nurses in a branch;
Figure 19 illustrates a calendar window
Figure 20 illustrates a task-editing window;
Figure 21 illustrates a task-adding window;
Figure 22 illustrates nurse site functionality;

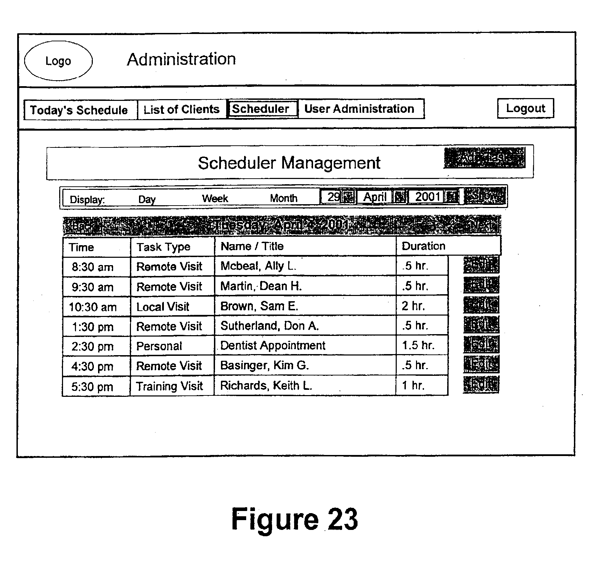

Figure 23 illustrates a nurse scheduler;

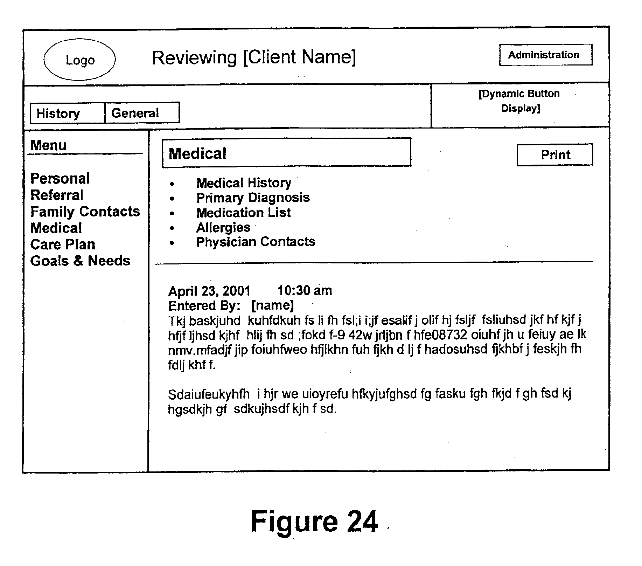

Figure 24 illustrates a client review interface;

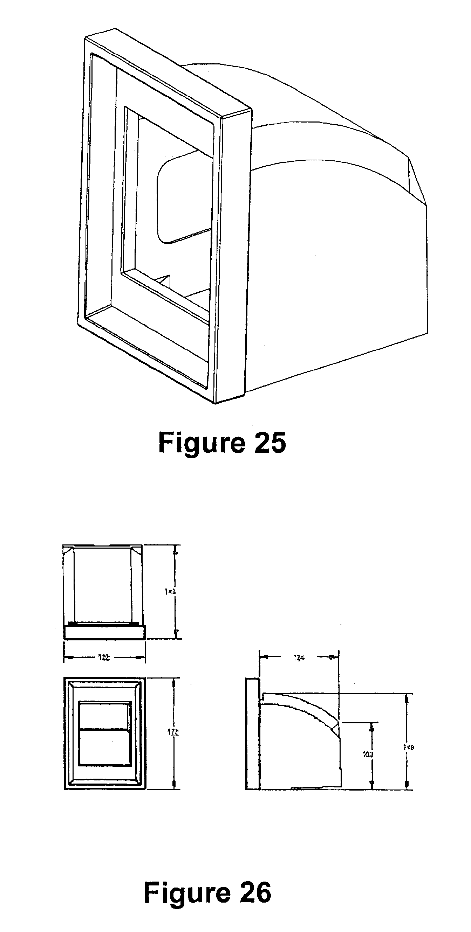

Figure 25 illustrates an isometric view of the camera enclosure;
Figure 26 a, b and c illustrate a top plan view, side elevation view and front elevation view of the camera enclosure; and

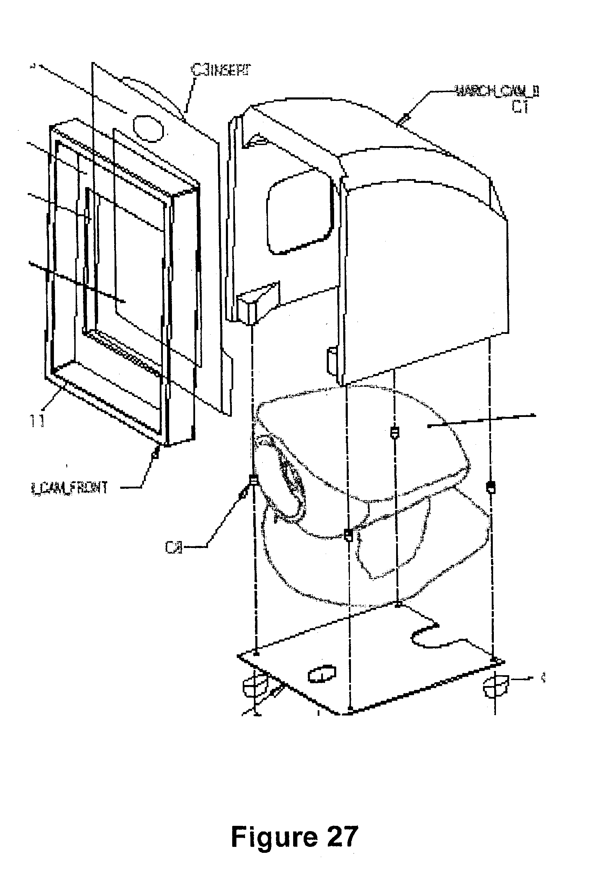

Figure 27 illustrates an exploded isometric view of the camera enclosure.

### Detailed Description of the Presently Preferred Embodiment

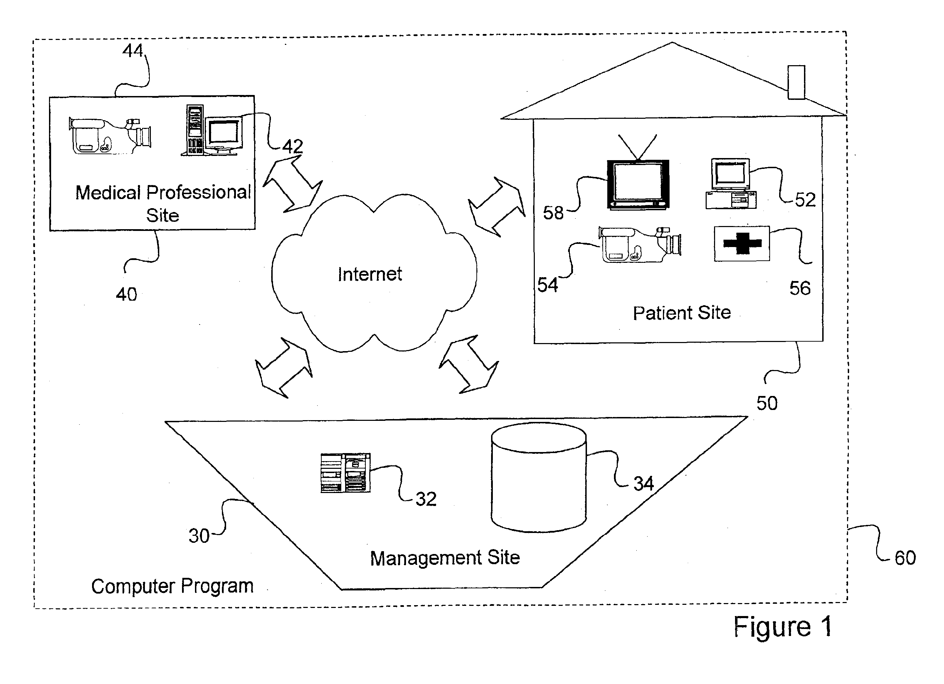

The present invention is directed to a remote health-monitoring system and method. As illustrated in Figure 1, the system includes a management site 30, at least one medical professional site 40, a patient site 50, and a computer program 60 operative to facilitate communications between the management site 30, the medical professional site 40, and the patient site 50 to provide remote health monitoring.

The management site 30 has a remote health-monitoring application server 32 and a database 34 accessible by the application server 32. The medical professional site 40 has a client computer 42 and a first videoconference camera 44 in communication with the client computer 42. The patient site 50 has a gateway computer 52, a second videoconference camera 54 in communication with the gateway computer 52 for relaying images of a patient, a medical Kit 56 having physiological data measuring devices in communication with the gateway computer 52 for relaying a patient's physiological measurements, and a television 58 for viewing informational displays, in an embodiment of the present invention, the medical kit is in wireless communication with the gateway computer 52.

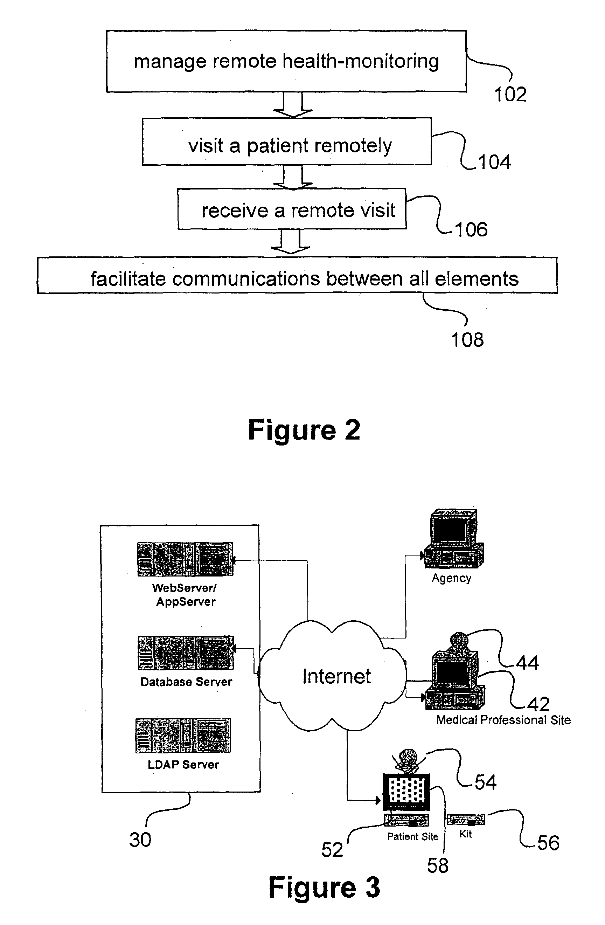

As illustrated in Figure 2, the method includes the steps of managing remote health-monitoring using a remote health-monitoring application server; and a database accessible by the application server, visiting a patient remotely using a client computer and a first videoconference camera in communication with the client computer, receiving a remote visit using a gateway computer 52, a second videoconference camera in communication with the gateway computer 52 for relaying images of a patient, a medical kit having physiological data measuring devices in communication with the gateway computer 52 for relaying a patent's physiological measurements, and a television for viewing informational displays, and facilitating communications between all elements to provide remote health monitoring using a computer program.

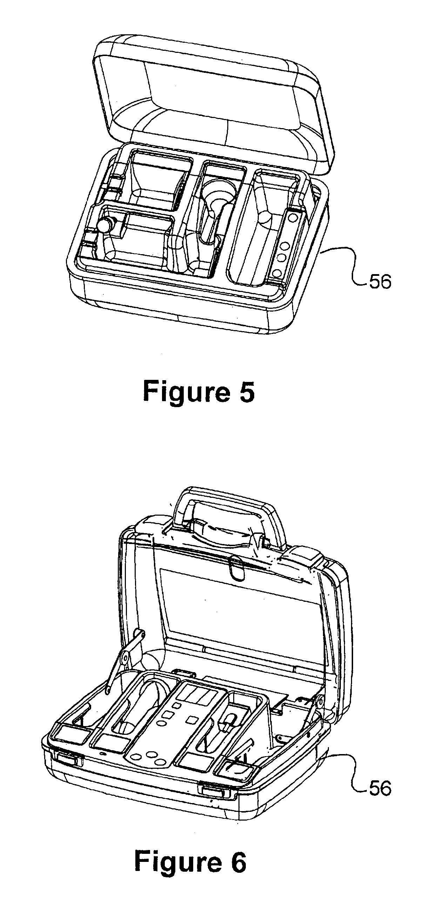

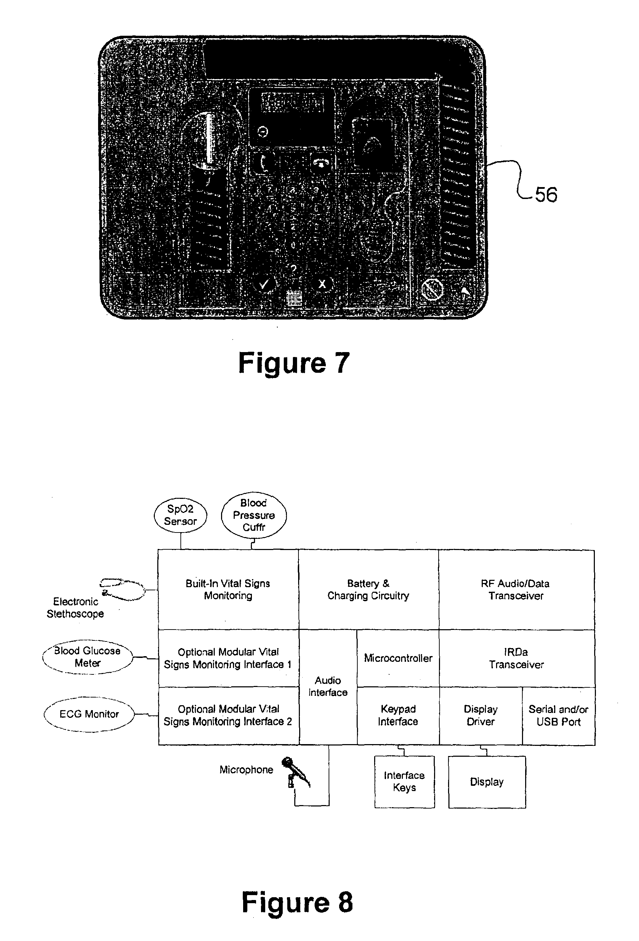

Figures 5 and 6 illustrate embodiments of the kit, whose architecture is further highlighted in Figure 8. The vital sign monitoring circuitry typically performs three standard vital sign measurement functions: Body temperature through a temperature probe, blood oxygen saturation level through an SpO2 sensor, heart and lung sound monitoring through an electronic stethoscope, and blood pressure through a blood pressure cuff that is automatically inflated through an onboard pump.

The medical kit is battery powered, rugged, portable, and has integrated cable management for the medical sensors, and in a preferred embodiment, supports configuration options in the choice of medical sensors installed as modular components. The medical kit includes several vital sign monitoring capabilities in a rugged and portable enclosure. Storage for the medical sensors and sensor leads are provided within the case, thereby eliminating clutter, improving ease of use, and adding to the units portability. Vital sign monitoring capabilities typically will include a temperature sensor, blood pressure cuff, SpO2 (blood oxygen) sensor, and stethoscope, and can be expanded to include other medical sensors. In one embodiment of the present invention, the system supports modular vital sign monitoring capabilities to enable the unit to be configured according to a particular patient's needs by including the specific medical sensors required for their health monitoring needs configured as requited, such as ECG leads.

The medical kit is used in conjunction with the gateway computer 52 52 that links the medical kit to a controlling computer and database at the management site. The medical kit remains at the home of the client whose health is being monitored, and can be stowed away until vital signs are actually being measured. It can then be easily brought out by a client to initiate a visit, and medical sensors applied. The battery generally provides over 12 hours of continuous device use before needing to be recharged, and is preferable of a sealed lead acid or a NiMh variety for long life.

The PTZ (Pan/Tilt/Zoom) video camera is housed in a video camera enclosure that provides protection to the camera, and provides a measure of privacy to the user when the camera is not in use by disguising the camera as a picture frame, enabling the camera to blend into its surroundings. The enclosure provides protection against damage to the video camera, which can be an expensive and delicate system component. The camera is protected from shock during transportation, or in the event that it is dropped. Certain cameras have pan and tilt mechanisms that allow the camera to be panned from side to side and tilted up and down. The mechanism is delicate and can be easily damaged if the unit is manually rotated or tilted, for instance if a child were to grab it and move it with their hands. The enclosure prevents this from accurring by protecting the camera with a clear glass plate in front and solid walls on all other sides, preventing a person from physically contacting the camera itself. The glass plate can be substituted with a plastic one, or other clear material.

Since the camera is disguised as a picture frame when the shutter is closed, it looks appropriate in virtually any location. Therefore, the camera can be on a table or bookshelf, enabling the unit to be positioned/located at an optimal distance from the user while in use with the shutter open, and remain there when not in use with the shutter closed. Since the camera is wired, it allows it to be made unobtrusive when not in use, without having to physically move it. The enclosure can be sized to house the largest PTZ cameras, providing optimum quality video capture, or miniaturized to hold smaller cameras without any loss in its effectiveness in hiding the camera or providing privacy when not in use, or it can be built as a "one-size-fits-most" device to support most any size of video camera, from high-quality PTZ models to smaller, more inexpensive units.

In one embodiment, the shutter is implemented as a clear piece of plastic that is folded in half. A 4"x6" picture is then inserted into the shutter, sandwiched between the two folded sides of the sheet. This enables a picture to be inserted and removed without the use of any adhesives. The shutter can be made from material other than plastic, be of any size, and can hide the camera with items other than pictures. When the shutter is open, the camera can focus on the user through the clear pane, providing higher image quality than those of pinhole cameras.

The use of a camera in the home can raise user concerns over privacy and discretion. Having an exposed video camera, even when turned off, may raise the concern that the user is being monitored continuously. By providing a shutter, in the form of a picture holder, the user can raise or lower the shutter to control when the camera can be used, providing an added element of control for the user. When the shutter is closed, the unit looks like a picture frame and completely hides the camera enclosed within, enabling the camera to "vanish" when not in use without having to physically move the unit. To visitors in the home, it is virtually undetectable as a camera

A nurse/patient videoconference enables real-time two-way interaction. A "store and forward" mechanism is provided for capturing data on the gateway computer 52, and then transferring it to the medical professional site. In this way, high quality digital still images and stethoscope recordings are captured.

During remote visits, a nurse controls the progress of measurements based on visual cues and verbal confirmations from the client/patient. During what is called an "unassisted measurement", measurements can be taken without nurse involvement With instructions typically given on the TV screen, the client follows a sequence prescribed by a remote application driven through the gateway, and is guided step-by-step using key presses on the kit.

A nurse making an in-person visit to the client can bring a laptop or PDA to the residence. In such a visit that device becomes a controlling host and will connect to the gateway computer 52, from which the management site and medical kit can be accessed. In this way, the nurse can conduct an in-person visit in a similar manner to a remote one.

The medical kit provides transmission of vital sign data and audio, such as stethoscope or integrated microphone input to a computer, set top box, or PDA. The device is battery powered and communicates with a host wirelessly allowing it to operate without cables, with the medical sensors and sensor leads stored inside the kit.

Vital sign measurements can be triggered remotely from a host through an RF Data Transceiver that receives commands to initiate or terminate measurements and transmits data back to the host to report on the status of measurements in progress and the results of the vital sign measurements once completed. In prototypes, a proprietary 900MHz transmitter was used as the RF data transceiver, however other RF transmission methods could be used. In the preferred embodiment, Bluetooth RF transmission and reception is used for both audio and data transmissions.

The audio mixer takes audio signals from either a microphone or an electronic stethoscope, as selected via a command from the host, and transmits these same signals to the host via an RF audio transmitter. In prototypes, a proprietary 900MHz transmitter was used as the RF audio transmitter, however other RF transmissions methods could be used. The micro-controller interprets commands received from the host to trigger vital sign measurements or select between one of the two audio sources, typically microphone and stethoscope. It also monitors the interface keys and sends a command to the host when any of the keys has been pressed. In this embodiment, two keys are implemented. A start call key is pressed when the user wants to initiate a video conferencing call to the nurse, and an end call key is pressed to end the call.

As illustrated in Figure 7, in an embodiment of the present invention, the kit includes configurable physiological data measurement options such as an interchangeable blood glucose meter, spirometer, and ECG module shown along with fixed stethoscope, blood oxygen, and blood pressure measurement capabilities. This allows the inclusion of a configurable and modular vital sign measurement capability. The system allows these capabilities to be expanded on through one or more slots or interfaces that can accept additional physiological data measurement functions in the form of plug-in modules, uniquely configured to suit an individual patient's particular medical condition. These optional slots can be implemented In a variety of ways, but a preferred embodiment uses the industry standard USB (Universal Serial Bus) electrical specification with a proprietary interface cable adaptor or "dongle" for each optional device supported.

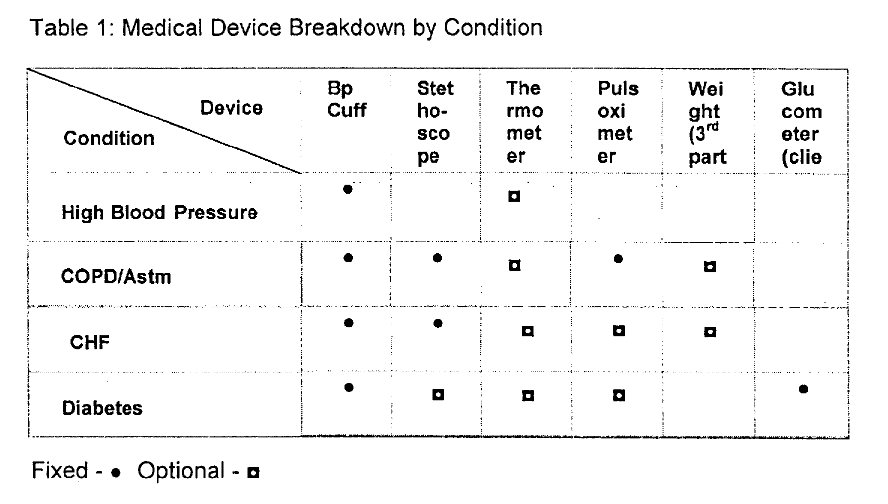

The USB implementation integrates RF (radio frequency) data transceiver components and RF audio transmitter components into one integrated RF transmitter incorporating the capabilities of both data and audio, and transmission and reception in one transceiver. This implementation utilizes the Bluetooth protocol and chipsets for this function, however other RF technologies could be employed.

An IRDa (Infrared Data Association) transceiver and wired universal serial port provide methods of communicating with the medical kit when the use of the RF transceiver would be undesirable, such as in cases where the medical kit is used in an aircraft where the use of RF devices is prohibited. The IRDa port adheres to the industry standard IRDa protocol, enabling the unit to be used with any IRDa capable device, such as a PDA or laptop. This permits the device to be operated remotely from up to six feet away using infrared protocol transmission. When a wired connection is desired, the USB port will provide this capability, allowing the device to be connected to any USB-capable host.

An optional display can be connected to the display driver to enable the medical kit to be used independently of a gateway computer 52, allowing vital sign measurements and status to be displayed locally on the attached display without requiting the use of a gateway computer 52. The nurse is able to interactively control the PTZ camera in the home, including panning, tilting, and zooming. Focus and iris control is provided local only. Care has been taken to account for command latency so that the nurse does not "overshoot" when adjusting the camera due to the delayed visual feedback to adjustments.

The management site provides a repository of all visit and client data, as well as an administration and management. It enables clients to interact with the system by serving up web pages that constitute the various client applications that it must support.

As described above, the nurse has the ability to direct the gateway computer 52 to capture high-resolution still images, or full-motion video clips, and/or high fidelity stethoscope audio data. In all capturing scenarios, it is desirable to have the real time conference continue, therein ensuring only minimal disruption in the normal nurse/client interaction. For certain types of medical consults, the entire visit can be recorded, thus capturing the entire videoconference, audio and video, for later review.

To capture video, the nurse will again position the camera and begin the capture. When enough data has been captured, up to 30 seconds worth, the capture time is limited only by storage, such as could be more than 30 seconds worth. the capture can be stopped. This capture will be local only and depend on the available bandwidth, as it would be impractical to transfer such a large amount of data from the gateway computer 52 to the nurse station in a store-and-forward mechanism.

To capture still images, the nurse directs the client and positions the camera, and once the camera is in the desired location, tells the Gateway to capture the image. This image can be captured at a higher resolution or quality than the normal video conferencing, particularly if this is over a low bandwidth channel, so there may be a small disruption in conferencing video if the capture is changed to a different resolution and quality. The image is captured on the gateway and forwarded to the nurse station.

To capture video, the nurse will again position the camera and begin the capture. When enough data has been captured, up to 30 seconds worth, the capture can pe stopped. This capture will be local only and depend on the available bandwidth, as it would be impractical to transfer such a large amount of data from the Gateway to the nurse station in a store-and-forward mechanism.

The transfer of still images and recordings should be done during the visit for the following reasons. If the connection is via broadband, the transfer is likely to be quick and will not be an inconvenience. If it is over POTS, then the client should be aware that the visit is still in progress and that they must wait for the transfer to finish. Otherwise, they might turn off the TV. pick up their phone and cancel the connection with the nurse site. The simplest behavior is to have the nurse and client wait for the transfer to finish after each capture before proceeding to the next step of the visit. Needing to be aware that transfers are going on "in the background" and having to wait for them to complete before the visit can be ended may be too obscure

A "Remote Data Recording In Visit" application enables a nurse to take a client's vital signs remotely through the Internet during a remote visit. Its functionality is closely related to the functionality of the Gateway as well as the functionality of the Kit. A wizard-like UI provides detailed instructions that allow a nurse to fully control the process of getting the vital signs through the gateway computer 52 and the medical Kit. The application will provide feedback on device error, network failure, inability to successfully get a device reading and processing status to the nurse as well as provide further instructions to correct the problem. The Application ensures the storing of only one set of data for a visit, but allows multiple visits for a client within the same day.

This application can present a specific UI to different clients based on their profile and location setting. For example, the system can be configured so that the stethoscope screen won't show up during an unassisted remote visit, or weight scale screen won't show up if a client doesn't have a weight scale in their profile.

If there are new, wired medical devices for the medical kit or new wireless medical devices for the gateway computer 52, there may be a need to upgrade the existing system software, adding additional schemas to the existing database or new layouts to the user interfaces (UI). The upgrade process is smooth, safe and done with minimal service disruption, such as at night.

Features include a wizard-like UI that provides detailed instructions and steps for a nurse to guide a patient through the data recording process remotely, feedback for results, and status and error messages. The system supports multiple visits per client per day, set separate vital sign profiles for each client, and each embodiment of the system will support a pre-defined set of medical devices.

The system allows a nurse to write progress notes during a remote visit and save it in the system's central database. Exception reporting is set by a nurse for acceptable range of vital sign measurement readings for a particular client, exception reporting can highlight those vital readings that are beyond the normal scope for a particular client.

The system captures sound tracks, still pictures, and short video clips for visually assessing the physical condition and behavioral indicators of a client and saves them as resource files in the system to allow future access; all the while keeping normal interaction with the nurse.

Commands issued from the nurse, vital sign readings coming back from the gateway computer 52, as well as data and resource files submitted to the database and accessed by the nurses are all encrypted, ensuring data secrecy, authentication and integrity. All access to a client's record is logged for audit purpose. All interaction between a client and a nurse during remote visit is logged

Offline data is the data submitted to the system database by the client during a client's unassisted remote visit, or offline monitoring. The database will provide a temporary storage place to hold this offline data until a nurse comes to review them and incorporate them into that client's vital sign records. In order to notify a nurse that there is offline data pending for processing, the system includes an inbox for a place to hold notifications or events sent to a specific nurse. For example, there are some offline vital signs data for Ms. Jones in the database pending to review. Another client Ms. Betty's offline vital signs data record shows she had an exceptional high Blood Pressure reading recorded yesterday which was 180/120; Ms. Betty also forgot to take the last pill last night which she should take three times a day. The Inbox works like an email inbox, allowing a nurse to login and check messages.

By default, all messages or notifications generated by clients will be sent to their primary nurse only. But nurses or administrators can forward the messages from their client to another nurse's in box. In this case, both nurses can view the same message but only one of them can mark it as "handled", For example, Nurse Rose will be on vacation next week; she wants to let nurse Jones handle all her clients' cases for her during her vacation. She activates her inbox's forwarding functionality and selects nurse Jones as the forwarding target. A copy of all her client's messages and notifications will then be forwarded to nurse Jones' inbox. Nurse Jones now has the privilege to receive and handle these messages and mark them as "handled" after they have been done. When nurse Rose comes back from her holiday, she can log into her inbox to review the messages handled by nurse Jones. She can then deactivate the forwarding functionality, wherein all her client's messages will be sent to her only again.

When a nurse logs in to the system, it will show how many new "messages" there are in the inbox and provide a link to go inside to check the detailed contents of the messages. The nurse can read them right away or ignore them and go ahead with other tasks, such as a remote visit or writing notes, and then come back to review messages in the inbox later. A nurse can mark a message as handled. After the message's "flag" has been changed to handled, it won't show up as a new message. Messages will then be auto-handled based on other activity in the system where possible, otherwise they are manually marked as "handled". Management of old messages in the inbox is manual. A warning can appear if too many old messages accumulate in the inbox to encourage the nurse to delete or archive old messages.

The system can include a feature called "Client Side Offline Data Retrieving" that is a functionality of the gateway computer 52. A typical use case for client side offline data retrieving would be: Ms. Jones is doing an unassisted visit. First, the gateway computer 52 knows Ms. Jones is using it, and then the Gateway goes to the system to find the "Offline Data Retrieving" profile for Ms. Jones, and knows that Ms. Jones should take BP and SPO2. It then first shows instructions to take BP and guides Ms. Jones through all the necessary steps, if the process succeeds, then the BP reading will be stored in the gateway computer 52 locally and temporarily. If it fails, the reading will be invalid and an error code generated. The patient can then choose to retry the BP up to 3 times. If it still fails, implying that something is probably wrong, the gateway computer 52 will go forward to take the SPO2. The same procedure happens to SPO2.

After Ms. Jones finishes SPO2, the application will show her offline vital sign readings like "BP: 120/90, SPO2: 98, do you want to submit the result?" or if there is an error code for BP, the message will say "You can not correctly measure your BP, do you want to notify the nurse?" If Ms. Jones presses "Submit" button, she will see "A notification has been submitted to the nurse successfully!" and the offline vital sign data goes to the system's central temporary storage. If there is an error code for BP or there is some exception reading, when the nurse logs on to her system, a notification or message will be placed in the inbox as described above. If the submission failed, it will show the reason why it failed and tell the client what to do. In Ms Jones' case, it might display "Network connection is down, try again later or at your next appointment at 7:15pm".

The system includes reviewing and incorporating offline data. For example, nurse Rose logs on to the Application, and her Inbox shows that she has 2 new messages. The first one is that "Ms. Jones took an unassisted visit yesterday and submitted some offline data in the database temporary storage". She leaves the Inbox and opens Ms. Jones' profile. She sees the "Review Offline Data" button is flashing and clicks it. The screen shows all the offline vital sign data Ms. Jones took by herself in an unassisted remote visit yesterday in a table, wherein she carefully reviews all the records.

All the records appear to be good except that there is an exception record icon in the second blood pressure that Jones took, which is 50/45. Rose knows that this is a bad reading, so she checks all the check boxes beside each reading except the 50/45 pair, then presses "Add to Client's Record" button, the screen reminds the nurse that data has been successfully added to Ms. Jones record. Rose selects the "Vital Signs History" tab. The screen shows Ms. Jones' vital data in both table mode and chart mode, all the data Rose submitted is there and marked as "offline measurements". Rose then goes back to the Inbox, where she marks the message as "handled".

The process of marking a message or a notification as "handled" may be better handled manually, since after a nurse reads a message or a notification from the Inbox, it likely should be up to the nurse to decide whether the message has been "handled" or not For example, when nurse Rose receives a notification that client Jane didn't take her pills three times yesterday as prescribed, she has to call her to confirm and tell her to take the pills today. Only after that can she go to the Inbox and mark this notification as "handled".

The individual access rights or permissions that are assigned to a user of the system determine the activities that that user can perform. It is easier to manage these permissions in an application if one defines a set of roles based on job functions and assign each role the permissions that apply to that job. It is then a simple matter of moving users between roles, rather than having to manage permissions for each individual user. If the function of a job changes, it is easier to simply change the permissions once for the role and have the changes applied automatically to all members of the role.

It is highly desirable to limit any access to health information to those employees who have a business need to access it. With this in mind, the following roles express exemplary examples of user accounts within the system. An administrator can easily assign these roles to users, and to assign multiple roles to a user in cases where it makes sense.

**Table 2:**

| User Roles and Descriptions | |
|---|---|
| Role | Description |
| System Administrator | Manages agencies and system wide policies |
| Agency Administrator | Manages branches, defines agency policy |
| Branch Administrator | Defines branch policy, manages staff accounts, and grants permissions to client medical records to staff |
| Staff | Can review and update their patients' medical records, schedule appointments with them, and conduct remote visits |
| Collaborator | Has restricted and time limited access to one or more medical records |
| Maintenance Personnel | Has access to the monitoring interface that give an overview of the health of the system. Can review the details of any problems, and track the performance of the system over time. Can perform system maintenance activities. |

An administrator is responsible not only for assigning roles to users, but also for the assignment of specific access rights to resources. For example, nurses assigned to a Staff role might not necessarily have access to the same patient medical records. It is up to an administrator to determine exactly which staff members can access what medical records. System administrators have the highest level of access. They can manage all user account types, assign roles to users, and give users access rights to resources, but cannot access actual client medical records. This role's major responsibility, however, is to manage agencies. In new installations, the system administrator will create and configure the system's first agency. Until the system administrator performs this task, no other useful work can be performed. The system administrator will then create an agency administrator account and hand over the login credentials to the responsible agency administrator.

The agency administrator can now begin to set the landscape for the agency. Branches and branch administrative accounts are created, and login credentials are distributed to appropriate personnel. Next, the agency administrator can decide what special services the agency will support. For example, will the 'Care-On-Demand' service and the 'Outsourced Scheduled Remote Visits' service be turned on? If so, the administrator needs to designate which branches within the agency can be providers of these services.

Branch administrators are responsible for the day-to-day activities associated with running a branch. These activities include inventory management, staff management, client management, scheduling, branch policy, and reporting.

Inventory management includes the management of both new and existing inventory, maintaining maintenance records, tracking equipment location, designating whether a gateway computer 52 and medical kit can support multiple user dwellings. As well, inventory management includes assigning equipment to any patient in a branch. If a gateway computer 52 is designated as multiple user dwelling capable, the branch administrator can assign and un-assign as many clients as appropriate to the gateway computer 52.

Staff management includes managing staff accounts and distributing login credentials to appropriate personnel, and maintaining current status of staff accounts, active or inactive. A branch administrator might render a staff account inactive as a result of an employee's termination or suspension. Staff management further includes assigning staff access rights to client medial records, granting care-on-demand privileges to staff, managing collaborator accounts, and managing staff training accounts.

Client management includes managing client accounts, reviewing and updating client medical records, maintaining current status of clients (active or inactive). A branch administrator would render a client account inactive if that client is not to receive further care. Even though appropriate staff can still review this medical record, no one will be permitted to modify the record in any way. While upcoming visits scheduled with inactive clients won't be removed from a nurse's schedule, any further visits will be disallowed. The nurse will also be prevented from scheduling new appointments with inactive patients. Client management further includes archives patient records, purging patient records, transferring patients to other branches, and managing client training accounts.

Scheduling involves any type of activity with staff, including remote visits with branch patients and those of branches to whom it is an Outsourced Scheduled Remote visits (OSRV) provider. The tool used for this activity is the master scheduler, Branch-policy includes selecting its 'Care-On-Demand' (COD) provider amongst ones designated by the agency administrator, configuring COD service behavior, and selecting its OSRV provider amongst ones designated by the agency administrator. It should be noted that a branch couldn't decide for itself if it can provide COD and OSRV services or not. It is the agency administrator that decides this. Reporting includes generating branch-specific reports, such as nurse activity reports.

Client management involves creating new patient records, reviewing and updating patient medical records, and maintaining current status of patients, as well as archiving patient records, purging patient records, and transferring patients to other branches. Branch staff are typically the ones providing direct or indirect care to patients, staff and nurse are used interchangeably. Their responsibilities mainly gravitate towards providing patient care and maintaining medical records. A complete list of their responsibilities follows.

Equipment assignments involve assigning equipment to patients. If a gateway is designated as multiple user dwelling capable, the nurse can assign, and un-assign, as many clients as appropriate to the gateway computer 52. Branch staff also review and maintain patient medical records, and can schedule any type of activity with a patient. However, they cannot schedule remote visits if their branch has outsourced this service, but can schedule work-related and personal activities. As well, they can participate in a scheduled remote visit with the nurse's patients, conduct local visits with the patients, receive only incoming requests for COD and can choose to partake in these visits if granted permission from the branch administrator, and initiate an impromptu or unscheduled visit, and can give client medical record access rights to collaborator accounts.

A collaborator, typically a referring physician or a specialist, can be given temporary access to a patient's medical records. It is the branch administrator who creates the account. Collaborators will have permanent accounts in the system, and will be given access by nurses to particular clients as required. Access to any given client's medical records will automatically expire after a period specified when the nurse gives access to the client record. Collaborators only have limited access to client records. Specifically, they cannot view'any notes marked as 'restricted' unless they are the authors of the restricted note. Collaborators can add notes of their own to a patient's medical record.

Maintenance personnel Keep the system running well. Maintenance personnel identify and resolve problems in the system by responding to problem alerts and monitoring system performance statistics and trends. They have access to detailed audit and log information that shows how the system is running and being used.

A patient is the actual client receiving medical care. The patient may or may not be given direct access to the system, such as for patient education, or the self-review of vital measurements. All patient medical data is logically considered as a single unit. That is, whether transferring a client to another branch, or archiving or purging a client from the system, all data associated with this client is affected. To ensure data integrity, confidentiality and availability, it is important to limit access to health information to those employees who have a business need to access it. The type of access control implemented in the system is a combination of role-based access and user-based access. As described earlier, an application user is first assigned a role by an administrator; it then, if necessary, further access to individual client medical records can be granted.

In order to identify suspect data access activities, the system will record all successful and unsuccessful login attempts into the system. The system can also log other data access activities in the context of a successful login into the application. A user identification system with password or a biometric identification system will be implemented to ensure that only authenticated users connect to the system. An automatic log off feature will also be implemented. Contents of the client record can be divided into two major areas: "Medical Record" and "Resource files". The "Medical Record" consists of a client's general information, visit and record history. "Resource files" are binary files taken in a visit, such as still pictures. The branch administrator creates general information with a client account. It provides a general overview about a client including several sub-categories and topics. Nurses with proper privileges have the ability to conveniently view, add, modify or delete it.

By default, nurses that belong to the staff role can only view basic information; no modification or deletion is permitted. All clients marked "Inactive" in status won't be shown. By default, only a branch administrator and a client's primary nurse can view and change general notes. General Notes fall into four subcategories: referral information, medical information, care plan, and goals and needs. By default, only a branch administrator and a client's primary nurse can view and change family information. It contains family contacts for a particular client. By default, only a branch administrator and a client's primary nurse can view and change physician information, which contains physician contact information for a particular client.

Visit and record history includes five sub-categories: "Vital Signs History", "Notes History", "Sounds", "Pictures" and "Video Clips". In general, this data is not editable. Filters can list all the exceptional records including vital sign readings, notes and other resource files when that exception happens. Vital signs history includes all the vital sign readings for a certain client. They can be reviewed in bath table and chart mode. Table mode is sorted by taken time in descending order. Exceptional readings will be highlighted. chart mode shows a patients vital sign readings in lines. It will support the following functionalities, such as different kinds of vital sign measurement combinations, show high, low exception threshold settings, show axis, show or hide grids, and show legend.

Time scrolling allows a nurse to view a small portion of a long period chart and move backwards and forwards flexibly. For example, a chart might contain one entire year's data but only show those for a specific month, and allow the nurse to view back and forth. An interpolating chart will automatically ignore null values and connect to the next available value; by default, the time unit is a day. If multiple measurements are taken in one day, the chart will show the average reading for that day and mark it so that later a user can zoom in to see all the readings in that day It is preferable for charts to be generated without extra tools or plug-ins, so that chart generation is seamless and doesn't inconvenience the user. In both Table and Chart mode, related vital sign measurements are shown together. For example, Systolic and Diastolic are shown together, as well as SPO2 and Pulse.

Notes history includes two types of notes: Progress Notes and Auxiliary Notes. Both are shown together sorted by recorded time in descending order, and a nurse with proper privileges is able to modify them. Progress Notes are taken when a nurse is visiting 9 client. The visit can be a remote visit or an in-person visit. The nurse can read or modify them during or after the visit. Auxiliary Notes are taken when a nurse is not visiting a client. They can be taken, read, added or modified at any time by a nurse with proper privileges.

Sounds include stethoscope readings recorded in different visits for a particular client. All sounds are sorted by "taken" time in descending order. If the nurse clicks one of the links, that particular sound track is played. Pictures include all still pictures taken in different visits for a particular client. All still picture links and captions are sorted by taken time in descending order. If the nurse clicks one of the links, that particular still image is shown in full size. Video clips include short video clips recorded in different visits for a particular client. All video clip links and captions are sorted by taken time in descending order. If the nurse clicks one of the links, that particular video clip will be played.

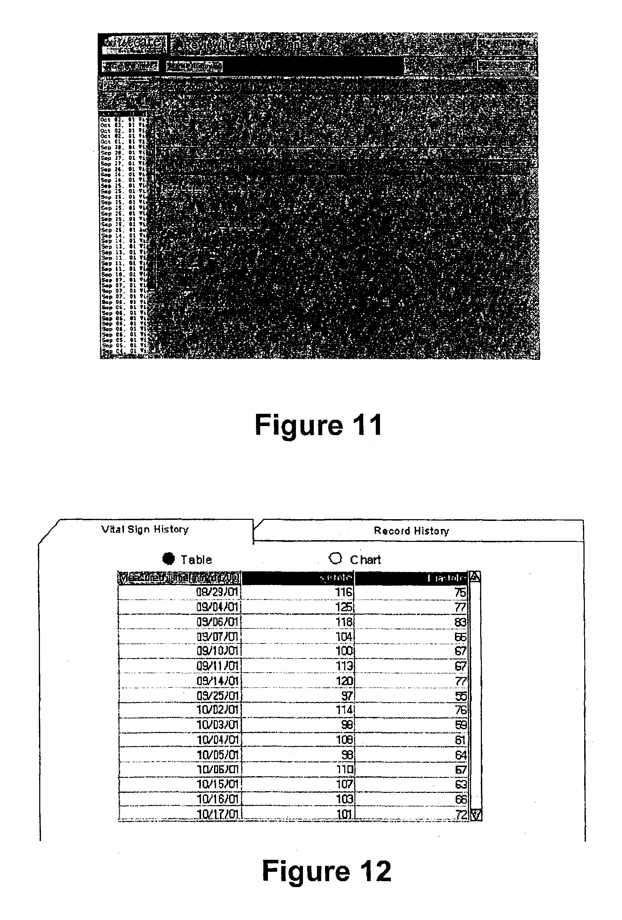

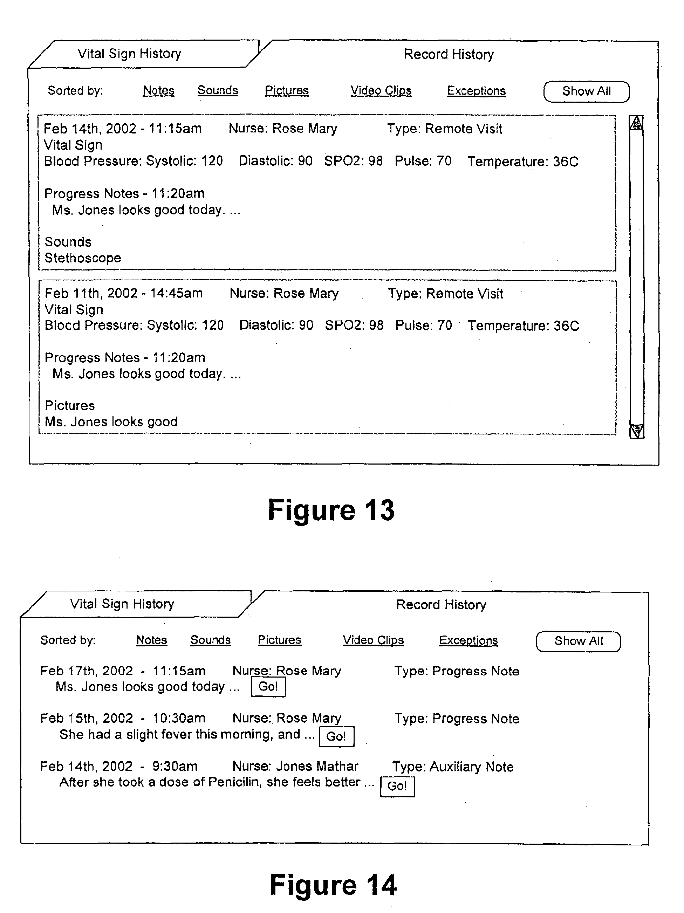

As time goes by, and a client's visit and record information becomes larger and larger, the information needs to be organized it so that nurses can readily find what they are looking for. Figures 11 and 12 illustrate an exemplary user interface (UI) layout for organizing all of the information. As illustrated in Figure 13, a "Vital Sign History" tab allows a nurse to toggle between Table Mode and Chart Mode to review all the vital sign readings for a particular client.

As illustrated in Figure 14, the "Record History" tab contains all medical records for a client, including vital sign readings, notes, sound tracks, still images and video clips. This record list can grow very long as time goes by, and sorting and classification mechanisms are provided to quickly locate a record. All records are classified by categories, such as "Notes", "Sounds", "Pictures", "Video Clips" and "Exceptions" and sorted by taken time in descending order. A "Show All" button is provided as the default selection for the "Record History" tab, which will show all medical records for a client in the same page context, and allow the nurse to compare different kinds of records in the same visit.

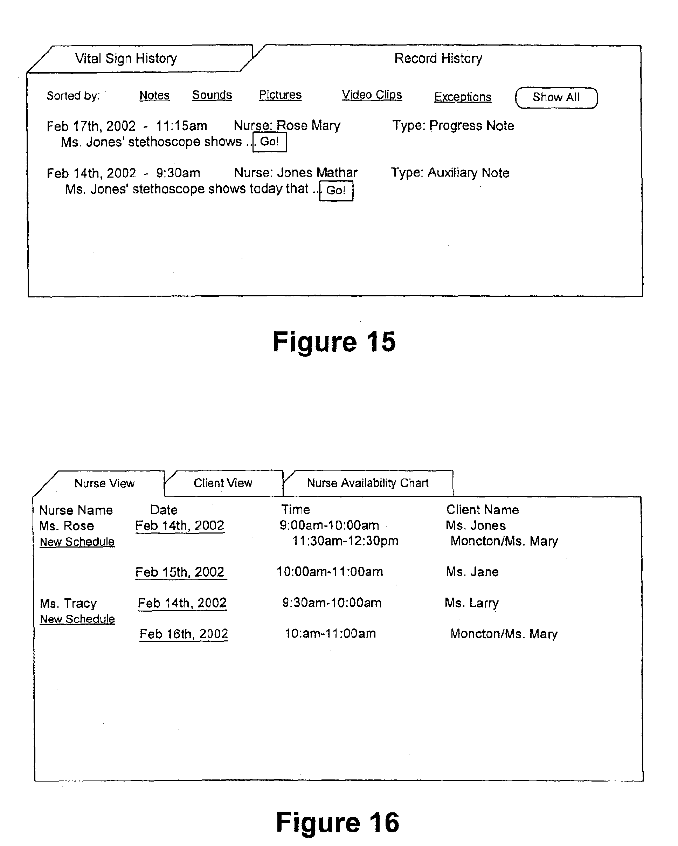

As illustrated in Figure 15, if a nurse wants to find a specific note quickly, she can use the classification mechanism and click 'Sorted by "Notes"', filtering out all other types of records. All notes will only show the first 30 characters in order to keep it short, and give the nurse brief clues. If the nurse finds the note she wants to review, she simply clicks the "Go" button and the page will direct her to details in the "Show All" context shown in Figure 14, so that the Sorted by Notes works like indexes and short cuts. As illustrated in Figure 16, the same approach applies to 'Sorted by "Sound"'. This page lists all the sound tracks and their captions, so that when the nurse selects one of them she will be brought to a certain record in the "Show All" context.

When working with client records, security is extremely important. This is true for both a clients medical records and resource files. Authorization control and disclosure tracking should comply with HIPAA (Health Insurance Portability and Accountability Act) standards. Security concerns fall into three separate areas: secrecy, authentication, and integrity. Secrecy means that an eavesdropper cannot intercept and understand messages. Authentication means that both sides are confident that they are talking to whom they think they are talking to. Integrity means that an interloper cannot modify messages in an undetectable fashion, even if they can't understand the contents.

For example, Alice the nurse wants to visit with Bob the patient, and the malicious hacker Charles is intent on cracking the system for his own nefarious purposes. All client records are protected, only authenticated and authorized users can get access to them. A typical use case might be: Ms. Jones' medical records, including her notes, vital sign readings, charts, still images, stethoscope sound tracks and short video clips, can only be viewed by her primary nurse Rose, not other nurses unless the administrator gives them privileges to do so.

Client record access is a single login process; only asking users for username and password once when they enter the system. When users try to get access to protected records, the system will let them in only if they have the proper privileges. All the connections that get access to protected records are encrypted, providing data secrecy, authentication and integrity as discussed earlier, and all access to the protected records will be logged for auditing.

Exception parameters are thresholds of acceptable vital sign measurements, or range of measurements, for a particular client. Some clients will have exception parameters set, some will not. Exception parameters are set once and used in Remote Visits, Care On Demand, and Offline Monitoring. There are two main scenarios to consider: setting exception parameters and exception notification. A nurse can add a new vital sign exception limit for a client and modify or delete it later. If an exception limit is set for certain vital sign measurements, all the exception items in that patient's vital sign readings are highlighted, both in Table mode and Chart mode, when displayed. Exception limits can include high or low threshold values or percentage deviation from the measurement trend.

In the case of both Remote Visit and Care On Demand, the Application will remind the nurse that an exception reading has been identified immediately when that measurement is taken. In the case of Offline Monitoring, when an exceptional vital sign record gets stored in the database, an exception notification message will be sent to the client's primary nurse's inbox. The next time that nurse logs into the system, the inbox will contain a notification message. The nurse can open the notification message and read the details, and once the message has been dealt with, it is marked as "handled".

The actual establishment of an audio/video link between the health care provider and the patient is a simple and efficient process. The complexities involved in establishing the link are hidden from the users while providing them with appropriate feedback. Nurses are provided with near real-time information relating to the current status of their patients. For example, if nurse A knows that a certain client has been waiting for Nurse B for over 3 minutes, nurse A might choose to take the call. Real-time status information will help alleviate visit delays and can even help to give patients of the system a more familiar feel to this technology, like using the telephone for instance.

As a nurse and patient prepare to engage in a videoconference, one of the parties will be ready for the conference before the other. The following connection scenarios are analyzed: The nurse can easily that the patient scheduled for 10 am hasn't yet pressed the 'Start Visit' button by looking at the real-time information displayed on the screen. At this point, the nurse may want to become available for a videoconference to the client. To accomplish this, the nurse selects the 10 am entry from the schedule. Hereafter, the context is clearly set when the client presses the 'Start Visit' button. A message such as "Welcome Mrs. Jones. I was expecting you, Please standby as we connect. Jane Murray" might be presented with a picture of Jane shown.

A patient is prevented from pressing the 'Start Visit' button before the nurse becomes available. In this situation, a slightly different message might be displayed. "Welcome Mrs. Jones. You are scheduled to visit Jane Murray at 10:00 am. Jane will be with you as soon as possible. Please standby. Should you require immediate assistance, you can press the 'Start Visit' button again to connect to the next available nurse. You can press your 'End Visit' button at any time to abandon this request The nurse in question is immediately notified that her "ten o'clock" is ready and waiting for the visit. The nurse can then choose to immediately acknowledge the notification and be connected with the patient, or spend some time reviewing that patient's records before proceeding to the visit.

By reviewing their real-time status information, other nurses with appropriate privileges can easily determine if someone else's patient is waiting for services and for how long. With this knowledge, this other nurse might decide to do the visit. A new message is displayed informing the patient of this development with a picture of Martha shown. "Hi Mrs. Jones. Jane Murray is running late. Can I visit with you instead? Martha Cooper." Press 'Start Visit' again if it's OK!

Patients initiate a scheduled remote visit by pressing the 'start visit' button on the medical kit. By pressing this button, a client is effectively giving permission to the remote care provider to engage in a videoconference. The patient can withdraw permission at anytime by pressing the 'end visit' button. Should the patient not be scheduled for a visit on the day the start visit button was pressed, a message will be displayed on the patient's television reminding them of their next scheduled visit, if any. If care-on-demand service is available to this patient, additional instructions can be presented.

Many variables need to be considered when generating a message presented to the patient, such as always wanting to providing information based on the entire context available. The algorithm used to generate messages considers whether the care-on-demand service is available and active, whether the patient has an approaching appointment, how long it is from the appointment's scheduled time, whether the patient has multiple appointments schedule on the day the start button was pressed, whether the patient has no appointments today, whether an unscheduled visit was initiated, and whether a scheduled visit was overridden by another nurse.

It is preferable to shield the nurses and client from the technology to enable them to get their job done. Video conferencing systems on the market today provide a means of connecting to another client by entering an IP address. With DHCP-based clients, this can become particularly difficult to set up since the IP addresses of the video conferencing end-points are constantly changing. The system's connection model hides this complexity from both nurses and clients, allowing them to connect to one another in a transparent fashion using their names alone.

The real-time status display and real-time connections described earlier facilitate a flexible scheduling scheme. Nurses da not need to do much rescheduling just because they're running a little early or late, or if they need to spend a little more time with any given patient. The schedule is similar to a receptionist's appointment book in a doctor's office where the doctor is not constrained by what is written in the appointment book. If both the doctor and the patient are ready for the appointment, the appointment takes place without a need to update the receptionist's appointment book.

Again, if a nurse's next scheduled client becomes available before the current visit is complete, this scheme can easily allow for another nurse with appropriate privileges to service that client Real-time connection feedback is provided to both the nurse and the patient. Comprehensive diagnostic information about the actual connection process is logged and made available for later analysis. This same log also contains commands and data received and sent during visits. Nurses can easily arrange to have an unplanned remote visit with any of their patients with no need to formally schedule an appointment. A nurse simply needs to initiate a manual call. A nurse can initiate a manual call by simply selecting the correct client from a list, then clicking on a 'Call' button, Should the patient press the 'Start Visit' button within a set time, the parties will be connected.

Patients are able to Initiate care-on-demand type remote visits in much the same way as ordinary scheduled remote visits. While the patient is waiting for care-on-demand, textual information will be displayed on the patient's television explaining that a nurse should be with them shortly. The patient, via training, documentation, or on-screen message, should be made aware that this service is not meant to replace traditional emergency health services.

Should a patient attempt a care-on-demand type visit and get no response within a predetermined amount of time, a note to that effect will be generated and placed in the patient's file. The note can include the call time and who was responsible to answer the call at that time. An appropriate message will also be displayed on the patient's television.

Agency and branch administrators are responsible for fine-tuning the exact behavior desired from the service. To begin with, each agency is given the ability to designate branches within their agency as outsourced 'care-on-demand' providers, if any. Next, individual branches select one of the designated care-on-demand providers as their provider. Finally, individual branches can then set their own care-on-demand policy. Note that this model could be simplified by allowing a special branch to be the only care-on-demand provider for the agency. But unless requested, the more flexible model described above is implemented. The branch itself handles all care-on-demand requests. All care-on-demand requests within the branch are forwarded to the outsourced care-on-demand provider for that agency. Care-on-demand requests made outside normal office hours will get forwarded to the outsourced care-on-demand provider, and care-on-demand calls are ignored.

Branch administrators can easily adjust their policy as necessary, including deactivating the service altogether. Individual nurses must be granted care-on-demand privileges to see incoming requests for service. As well, these nurses will have implicit access to medical records of all clients permitted to receive care-on-demand. When nurses review a patient's medical records, they can easily determine if data was collected from a care-on demand type remote visit Nurses with care-on-demand privileges must let the system know if they are currently available for calls. Reciprocally, the application will notify nurses of pending care-on-demand calls.

In an ideal world, a patient wishing care-on-demand would be connected immediately to a nurse. When the volume of calls exceeds the capacity to answer, queuing calls may become necessary. The Application ensures that all incoming care-on-demand calls are handled efficiently. As the care-on-demand call arrives and reaches the front of the queue, the system will choose the first nurse to present the call to, according to a set of rules, such as cyclic to next available nurse, sequential distribution, target nurse with least calls, or target longest idle nurse. More refined distribution methods can also consider related characteristics between nurse and patient; ethnic origins and language are examples of these. Unless directed otherwise, the simplest method will be implemented, namely a sequential distribution.

Typically, an agency administrator will supply the outsourced care-on-demand provider with nurse accounts to be used by its staff. A branch administrator account can also be given to the provider should they be allowed to manage their own user accounts. Like nurses of ordinary branches, members of the provider should also have access to client records for the branches they support, Access to these records can be implicitly granted when the agency designates a branch as the outsourced care-on-demand provider.

The visit scheduler provides two levels of activity planning: a master scheduler used by the management of a branch to review and schedule appointments for all nurses and patients, and an individual scheduler used by nurses for their personal schedule. The visit scheduler is web-based and secure to allow the user to use it either at work or at home. However, a health care provider can choose a proprietary scheduler or use their existing one. The scheduler is an activity manager and reminder like the other schedulers that nurses might currently use. Where appropriate, the system uses the information available from the scheduler to provide additional context to visits. For example, if a client presses the "Start Visit" button, the TV display can show when a pending appointment is to start and the nurse with whom the visit is scheduled. Or on the nurse side, the scheduler can provide hot links for the nurse to easily connect to pending visits.

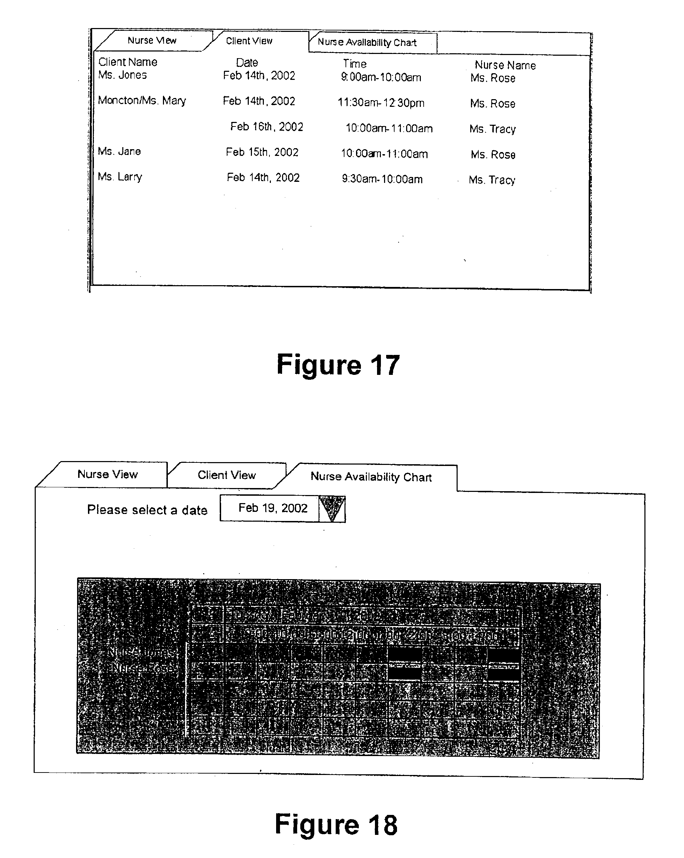

The master scheduler allows the management of a healthcare branch to control internal resources and activity as well as outsourced remote visiting activity- Key functionality includes: The branch administrator can track and manage, create, modify and delete, schedule entries for each nurse and client inside the branch. The administrator of an outsourcing remote visit provider can track and manage, create, modify and delete, and schedule entries for all the clients in the outsourced branch. The administrator of an outsourced branch can view schedule entries between clients in the branch and nurses in the outsourcing remote visit provider. Administrators can create schedules in a flexible way. For example, as illustrated in Figures 17 through 19, they can toggle between "Nurse View" and "Client View", and included is a "Nurse Availability chart" that shows all nurse activities in a certain day.

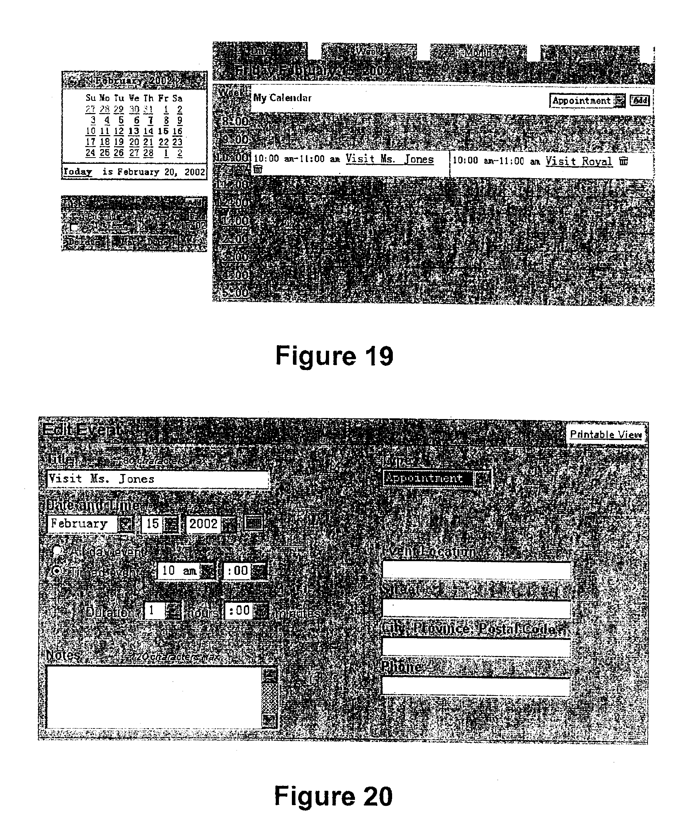

A nurse view shows all nurses' appointments in a branch and provides the ability to create, modify and delete a scheduled visit. As illustrated in Figure 17, if the "New Schedule" hyperlink next to Ms. Rose is clicked, it will direct administrators to the "Create Schedule Mode" in nurse Rose's individual scheduler, where they can pick a date and add a new appointment. As illustrated in Figure 18, the client view shows all the clients with whom a branch can make an appointment, including those from an outsourced branch. The administrator of an outsourced branch can use this view to see schedules between his clients and nurses of the outsourcing remote visit provider. As illustrated in Figure 19, the nurse availability chart shows the time availability status of all nurses in a branch. It allows the administrator to find out which nurse is available in a specific time slot.

In a typical use case for example, Mr. Joseph is an administrator of Branch X. He wants to make an appointment for client Ms. Jones. First, he takes a brief look at the "Nurse Availability chart" and finds out that Nurse Rose is available from 11:00am - 12:00pm on February 19. He returns to the "Nurse View" of the "Master Scheduler", clicks the "New Scheduler" link next to nurse Rose, and in nurse Rose's "Individual Scheduler" Mr. Joseph makes a new appointment with client Jones. Whether nurses, administrators or some combination control a nurse's schedule is up to the organization. The scheduling approach chosen is not mandated or enforced by the system.

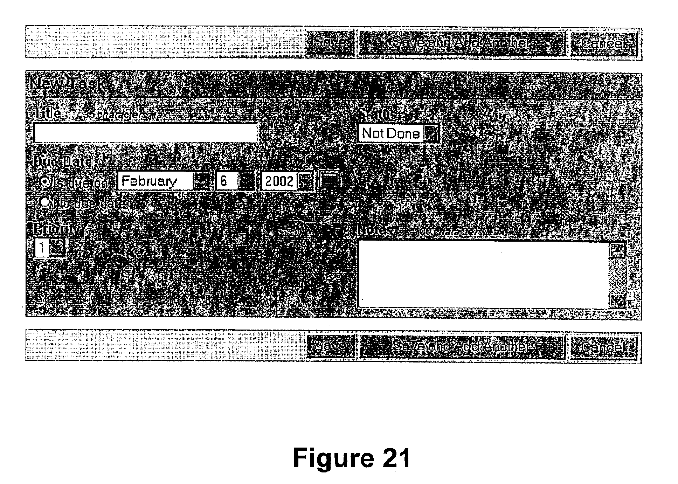

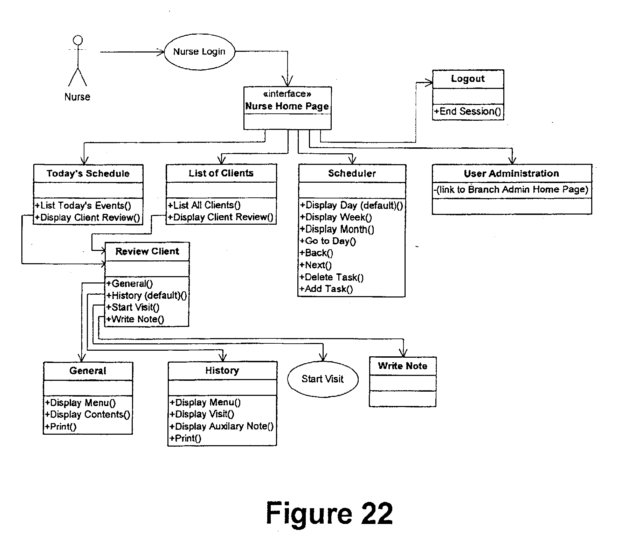

The individual scheduler is web-based and enables the nurse to fully and flexibly control her daily activity, includes a calendar, task and reminder, and supports multiple time zones. The calendar shows the entries of the individual scheduler. As illustrated in Figures 20 and 21, the individual scheduler can display the calendar in different views, such as day, week, month and year, to allow a nurse to view and modify existing appointments, and to create new schedules. As illustrated in Figure 22, the task window allows a nurse to input or modify a task name, due date, status, priority and notes. Tasks supply a way for nurses to track the progress of their work.

When a nurse adds a new appointment, it also sets the reminder automatically, which will notify the nurse and possibly the client ten minutes before the schedule. At any time, the nurse can go to "Reminder" to change the Reminder time and/or Repeat times. A "Support Multiple Time Zones" feature ensures that the same event will show the correct time in different time zones. On the nurse side, the event is a visual signal inside the nurse application to allow the nurse to click on it for more detail. In the client side, a visual attraction can be included to remind the client that an event is due. When the client turns on the TV, and presses the "Start Visit" button, the event detail will be shown on the TV.

Agencies that already have their own automated scheduler can still use their facilities, but inputting the same schedule information to the system will remind the client and nurse when the visit arrives. Otherwise, if the nurse doesn't use the scheduler in the system, her existing automatic reminder will notify her, but it is the nurse's responsibility to remind the client when a schedule is due. As described earlier, collaborators are typically referring physicians or specialists. They are given temporary access to a patient's medical records for reviewing purposes. They can also incorporate feedback into the patient's medical records in the form of notes. One of the many responsibilities of the branch administrator is to manage remote collaborator accounts in the branch. Management of collaborator accounts includes the creation and deletion of collaborator accounts, activation and deactivation of accounts, and the distribution of credentials to the collaborator,

Nurses also have responsibilities in regards to the management of collaborator accounts, including the assignment of patient medical record access rights to collaborator accounts, as well as the specifying of an expiry date to each access right given to collaborators. By default, the expiry date is set to two weeks past the initial grant date, and can be extended indefinitely. A branch administrator can also perform any of these activities.

As mentioned earlier, access to patient medial records by collaborators should be temporary in nature. Access to any given client's medical records will automatically expire after a period specified when the nurse gives access to the client record. Collaborators have limited access to client records, Specifically, they cannot view any notes marked as 'restricted' unless they authored the note. A collaborator logs into the system like any other user. Once the system validates the collaborator's credentials and confirms that the collaborator's account is active, the system displays a specialized presentation. The presentation gives them access to clients medical records assigned to them unless the access right has expired. Inactive client medical records are also excluded.

Unlike the interface presented to a nurse, the collaborator's interface does not provide scheduling and visiting capabilities. The interface is specialized for the review of patient medical records. The only other permissible activity is to add 'Collaborator' type notes to a patient's record. These notes are clearly labeled as 'Collaborator' type notes to all users capable of reviewing records. The system will autamatacally send a notification message to the primary care giver when a collaborator adds a note.

For applications where portability of vital sign monitoring capabilities is desirable, the invention provides a rugged, compact, and portable unit that allows vital sign monitoring capabilities to be carried around and deployed quickly and efficiently. As well, for the home health care market, this is particularly important as the devices are transported from one patient to the next after a term of care has been completed. For the home health care market, it is desirable that when the device is not in use, that its intended purpose is not evident when the medical kit is closed. This is provided in the design through an enclosure that looks like a conventional brief case or travel case. The medical kit provides integrated storage for both the sensors and their leads, providing enhanced device usability by avoiding tangled leads and providing clear indications to non-medical users which sensor corresponds to a desired vital sign measurement.

By designing the medical kit as a wireless peripheral, the medical kit can be used in a number of different applications. The medical kit may be used by a health care professional when it is used in conjunction with a desktop PC, laptop, or PDA. In one application as a peripheral in a home health care system, the medical kit can function as a peripheral to a set top box. In wireless device applications, the user can position the medical kit independently of the display device. The invention provides a system and method of monitoring a patient over the Internet via secure, non-dedicated connections that are less expensive and more flexible and scalable than the user of traditional dedicated lines to remote sites incorporating video, voice and data.

The management site manages connections efficiently by keeping a record of the current IP addresses for patient gateway computer 52s and nurse computers. Nurses need only deal with or remember client names while the application manages the underlying network connections between nurse and patient whenever it is required. When a nurse station or patient gateway computer 52 contacts the application server, the server validates these using what is known as a secure certificate to ensure that they are authorized users of the system- At the same time, the server records the IP addresses of all clients on the system in a table in the database. When a video conferencing connection or other network connection is required between a nurse and a patient or a nurse and another healthcare practitioner, the application server looks up the endpoints IP addresses from this table to initiate the connection. By providing this IP address lookup table, the system can support users with dynamically assigned IP addresses, or users accessing the system from different locations.

The invention can prove beneficial on aircraft and ships where the medical diagnosis of a passenger is carried out remotely for a variety of reasons, including the decision as to whether to continue on the planned route or divert to a closer airport or sea port. A teleconference visit is a convenience to the patient, especially for those patients who would have difficulty traveling to the doctor's office or hospital. A videoconference visit, connecting a nurse station to a patient station via a communications link, is more economical than a home visit, thereby providing both convenience and cost savings.

Although the present' invention has been described in considerable detail with reference to certain preferred embodiments thereof, other versions are possible. Therefore, the spirit and scope of the appended claims should not be limited to the description of the preferred embodiments contained herein.

## Claims

1. A remote health-monitoring system comprising:
a management site having:
a remote health-monitoring application server; and
a database accessible by the application server;
at least one medical professional site having:
a client computer; and
a first videoconference camera in communication with the client computer;
a patient site having:
a gateway computer;
a second videoconference camera in communication with the gateway computer for relaying images of a patient;
a medical kit having physiological data measuring devices in communication with the gateway computer for relaying a patient's physiological measurements; and
a television for viewing informational displays; and
a computer program operative to facilitate communications between the management site, the medical professional site, and the patient site to provide remote health monitoring.

2. The system according to claim 1, wherein the medical kit is in wireless communication with the gateway computer.

3. The system according to claim 1, further including an enclosure for housing the second teleconference camera to protect and disguise the camera when not in use, the enclosure comprising:
a six-sided box with one side being a removable panel through which the camera can be pointed.

4. The system according to claim 1, further including a portable client computer capable of connecting to the gateway to access both the medical kit and the management site to enable the conducting of in-person visits in a similar manner to that of remote visits.

5. The system according to claim 1, wherein the medical kit is a peripheral to a controlling device.

6. The system according to claim 1, wherein the medical kit is modular to provide interchangeable physiological data measuring devices.

7. A remote health-monitoring method comprising the steps of:
(i) managing remote health-monitoring using a remote health-monitoring application server; and a database accessible by the application server;
(ii) visiting a patient remotely using a client computer and a first videoconference camera in communication with the client computer;
(iii) receiving a remote visit using a gateway computer, a second videoconference camera in communication with the gateway computer for relaying images of a patient, a medical kit having physiological data measuring devices in communication with the gateway computer for relaying a patient's physiological measurements, and a television for viewing informational displays; and
(iv) facilitating communications between all elements to provide remote health monitoring using a computer program.

8. A remote health-monitoring system comprising:
means for managing remote health-monitoring using a remote health-monitoring application server, and a database accessible by the application server;
means for visiting a patient remotely using a client computer and a first videoconference camera in communication with the client computer;
means for receiving a remote visit using a gateway computer, a second videoconference camera in communication with the gateway computer for relaying images of a patient, a medical kit having physiological data measuring devices in communication with the gateway computer for relaying a patients physiological measurements, and a television for viewing informational displays; and
means for facilitating communications between all elements to provide remote health monitoring using a computer program.
